# EUROPEAN PATENT SPECIFICATION

(11) **EP 4 197 484 B1**
(45) Date of publication and mention of the grant of the patent: **21.05.2025**
(21) Application number: 22213738.2
(22) Date of filing: 15.12.2022
(51) Int. Cl.: A61C 1/14, A61B 17/16, A61C 1/12

(54) **DENTAL HANDPIECE**
ZAHNÄRZTLICHES HANDSTÜCK
PIÈCE À MAIN DENTAIRE

(30) Priority: 17.12.2021 JP 2021205568; 16.09.2022 JP 2022148345
(43) Date of publication of application: 21.06.2023
(60) Divisional application: 25166408.2
(73) Proprietor: Nakanishi Inc., Kanuma-shi, Tochigi 322-8666 (JP)
(72) Inventor: INOUE, Yamato, Tochigi, 3228666 (JP); IWAI, Yasuo, Tochigi, 3228666 (JP); OTSUKA, Hiroki, Tochigi, 3228666 (JP)
(74) Representative: Lewis Silkin LLP

(56) References cited:
- EP-A1- 0 421 907
- JP-A- H01 250 247
- US-A- 4 690 641
- US-A- 5 591 028

## Description

### TECHNICAL FIELD

The present disclosure relates to a dental handpiece.

### BACKGROUND ART

An air turbine handpiece that cuts an affected part by a rotational force of an air turbine, and a motor handpiece that cuts an affected part by a driving force of a motor are known as dental handpieces. Each of these dental handpieces holds a dental treatment tool by a chuck mechanism housed in a head portion, and cuts the affected part by rotating the dental treatment tool at a high speed.

A technique has been proposed in which by preventing a reduction in chucking force of a chuck mechanism that holds a dental treatment tool, dropping out, idling, and the like of the dental treatment tool are prevented (for example, JP3126887B and JP2006-122080A).

JP3126887B discloses a configuration in which the dental treatment tool (chucked member) is chucked by a ball that protrudes and retracts in a radial direction from a tubular chuck shaft.

JP2006-122080A discloses a holding mechanism including: a tubular tool holding member that holds the dental treatment tool; a tapered member that is disposed outside the tool holding member; and a coil spring that biases the tool holding member upward. Slits are formed at three locations along a circumferential direction of the tool holding member, and small pieces are fitted into the respective slits. An outer peripheral surface of each of the small pieces comes into contact with the tapered member when the tool holding member receives a biasing force from the coil spring, and each small piece is pushed inward in a radial direction due to a wedge effect of a tapered surface. As a result, each small piece engages with the dental treatment tool, and the dental treatment tool is held in the tool holding member.

However, in the chuck mechanism described in JP3126887B, the dental treatment tool is locally gripped by the ball, so that problems arise that wear easily occurs and a gripping force is easily reduced.

In JP2006-122080A, a gripping force on the dental treatment tool depends on a frictional force of the tapered surface. Therefore, when a particularly strong vibration is, for example, applied to the tapered surface during cutting, a gap may be created between the dental treatment tool and the small piece, and the dental treatment tool may drop out of the tool holding member. Since the tapered member is thin-walled, a gradient of a taper is shallow, and biting on the tapered surface easily occurs. As a result, attachment and detachment failures of the dental treatment tool easily occur.

Therefore, an object of the present disclosure is to provide a dental handpiece capable of improving wear resistance of a chuck mechanism and always stably holding a dental treatment tool regardless of a cutting condition.

US 4690641 discloses a chuck for receiving a plain shank of a dental burr. The chuck is rotatably mounted in the head of a dental handpiece and is driven by a pinion or by turbine vanes. The chuck comprises a sleeve adapted to receive the shank of a burr and provided with at least one elongate clamping claw which is movable radially between an inner clamping position and an outer releasing position. Facing grooves in the clamping claw and in an actuating member which is movable axially relative to the sleeve define a ramp way for a row of bearing balls. The ramp way extends in a direction which is generally axial but is inclined at an angle of 1 DEG to 4 DEG to the axis so that the clamping claw is moved radially by axial movement of the actuating member. The clamping claw is spring biased to a clamping position and is movable to releasing position by a push button for moving the actuating member axially relative to the sleeve and thereby moving the clamping claw radially outwardly.

JP H01 250247 and US 5,591,028 disclose a dental handpiece of the preamble of claim 1.

### SUMMARY

According to the present invention, there is provided a dental handpiece as defined in appended claim 1. Preferred features are set out in its dependent claims.

### BRIEF DESCRIPTION OF DRAWINGS

Fig. 1 is an overall view of a dental handpiece according to the present disclosure.
Fig. 2 is a schematic cross-sectional view of a head portion of the dental handpiece shown in Fig. 1.
Fig. 3 is a partially enlarged cross-sectional view showing a chuck mechanism housed in a sleeve.
Fig. 4 is an exploded view showing members constituting the chuck mechanism.
Fig. 5 is a perspective view of divided movable pieces.
Fig. 6 is a perspective view showing a state after the members shown in Fig. 4 are assembled.
Fig. 7 is a cross-sectional view taken along a line VII-VII shown in Fig. 3.
Fig. 8A is a view stepwisely illustrating a process of performing a chucking operation by inserting a dental treatment tool into the chuck mechanism.
Fig. 8B is a view stepwisely illustrating the process of performing the chucking operation by inserting the dental treatment tool into the chuck mechanism.
Fig. 8C is a view stepwisely illustrating the process of performing the chucking operation by inserting the dental treatment tool into the chuck mechanism.
Fig. 8D is a view stepwisely illustrating the process of performing the chucking operation by inserting the dental treatment tool into the chuck mechanism.
Fig. 9A is a view stepwisely illustrating an operation of releasing chucking of the dental treatment tool inserted into the chuck mechanism.
Fig. 9B is a view stepwisely illustrating the operation of releasing the chucking of the dental treatment tool inserted into the chuck mechanism.
Fig. 9C is a view stepwisely illustrating the operation of releasing the chucking of the dental treatment tool inserted into the chuck mechanism.
Fig. 9D is a view stepwisely illustrating the operation of releasing the chucking of the dental treatment tool inserted into the chuck mechanism.
Fig. 10A is a view stepwisely illustrating an operation of the chuck mechanism when an external force is applied to the chuck mechanism.
Fig. 10B is a view stepwisely illustrating the operation of the chuck mechanism when the external force is applied to the chuck mechanism.
Fig. 10C is a view stepwisely illustrating the operation of the chuck mechanism when the external force is applied to the chuck mechanism.
Fig. 11 is a schematic cross-sectional view showing a state where the dental treatment tool is held between the divided movable pieces.
Fig. 12 is a view illustrating a case where a taper angle of a first tapered surface and a second tapered surface is different from a taper angle of a third tapered surface and a fourth tapered surface.
Fig. 13 is a view illustrating a case where the taper angle of the first tapered surface and the second tapered surface is different from the taper angle of the third tapered surface and the fourth tapered surface.
Fig. 14 is a schematic cross-sectional view showing the divided movable pieces that each include the second tapered surface on an outer peripheral surface, and a cylindrical slide member that includes no first tapered surface on an inner peripheral surface.
Fig. 15 is a schematic cross-sectional view showing the divided movable pieces that each include no second tapered surface on the outer peripheral surface, and the cylindrical slide member that includes the first tapered surface on the inner peripheral surface.
Fig. 16 is a partial cross-sectional view of a chuck mechanism including a cylindrical fixing member that is divided into two parts.
Fig. 17 is a cross-sectional view showing a chuck mechanism according to a second modification that includes divided movable pieces whose axial length is extended.
Fig. 18 is a cross-sectional view showing a chuck mechanism according to a third modification in which each of protruding portions is formed integrally with a respective one of divided movable pieces.
Fig. 19 is a schematic cross-sectional view showing a simplified configuration of a chuck mechanism according to a fourth modification.
Fig. 20 is a schematic cross-sectional view showing another simplified configuration of a chuck mechanism according to a fifth modification.

### DESCRIPTION OF EMBODIMENTS

Hereinafter, embodiments of a dental handpiece according to the present disclosure will be described in detail with reference to the drawings. Here, a configuration example of an air turbine handpiece will be described, but a configuration of a motor handpiece or the like including another driving mechanism may be used.

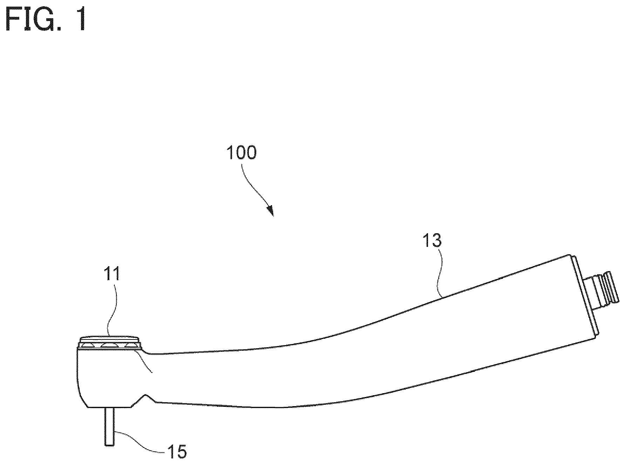

Fig. 1 is an overall view of a dental handpiece 100 according to the present disclosure.

The dental handpiece 100 includes a head portion 11 and a grip portion 13 that extends rearward from the head portion 11. The head portion 11 includes a chuck mechanism which will be described in detail later and in which a dental treatment tool 15 is mounted so as to be attachable and detachable, and rotatably drives the dental treatment tool 15 mounted in the chuck mechanism. In the configuration, a grip portion is provided with an air supply passage (not shown) and an exhaust passage (not shown) for compressed air, the dental treatment tool 15 is rotatably driven by the compressed air supplied from the air supply passage, and the compressed air used for driving is discharged through the exhaust passage.

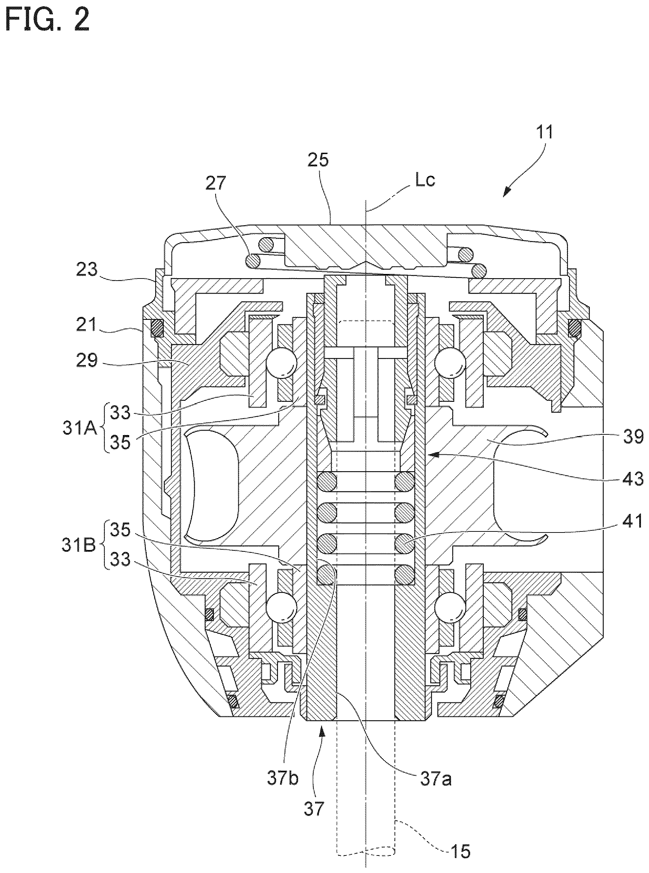

Fig. 2 is a schematic cross-sectional view of the head portion 11 of the dental handpiece 100 shown in Fig. 1. In the following description, an insertion and extraction direction of the dental treatment tool 15 when the dental treatment tool 15 is mounted in the head portion 11 is also referred to as an up and down direction or an insertion direction, a front in the insertion direction is also referred to as an upper side, and a rear in the insertion direction is also referred to as a lower side.

The head portion 11 is formed by being surrounded by: a head housing 21 that is connected to the grip portion 13 and covers an outer periphery of the head portion 11; a head cap 23 that covers an upper portion of the head housing 21 and is fixed to the head housing 21; and a push button 25 that covers a top of the head cap 23. A biasing spring 27 is provided between the push button 25 and the head cap 23, and the biasing spring 27 biases the push button 25 upward.

A cartridge case 29 is housed inside the head housing 21. The cartridge case 29 is fixed integrally with the head housing 21 by assembling the head cap 23 from above the head housing 21. Outer rings 33 of a pair of rolling bearings 31A and 31B are fixed to an inner peripheral side of the cartridge case 29. Inner rings 35 of the rolling bearings 31A and 31B are fitted with an outer peripheral surface of a hollow cylindrical sleeve 37.

The sleeve 37 is rotatably supported by the rolling bearings 31A and 31B, and the dental treatment tool 15 is inserted into the sleeve.

A rotor 39 is fixed between the rolling bearing 31A and the rolling bearing 31B on the sleeve 37. The rotor 39 is a rotating body including a turbine blade, generates a rotational force by the compressed air supplied into the cartridge case 29, and rotatably drives the sleeve 37. That is, the sleeve 37 is supported by the head housing 21 and the cartridge case 29 through the rolling bearings 31A and 31B, so that the sleeve 37 rotates with the rotational force generated by the rotor 39 as a drive source.

The sleeve 37 includes a small diameter portion 37a and a large diameter portion 37b on an inner peripheral surface thereof. A coil spring 41, which serves as an elastic biasing member that biases a cylindrical slide member 45 forward in the insertion direction of the dental treatment tool 15, is disposed at a lower end of the large diameter portion 37b. The coil spring 41 has an inner diameter that allows the dental treatment tool 15 to be inserted through the coil spring 41 along an axis Lc of the sleeve 37, and has a configuration in which space efficiency is improved in the sleeve 37. A chuck mechanism 43 to be described below includes the sleeve 37 and members housed in the large diameter portion 37b of the sleeve 37.

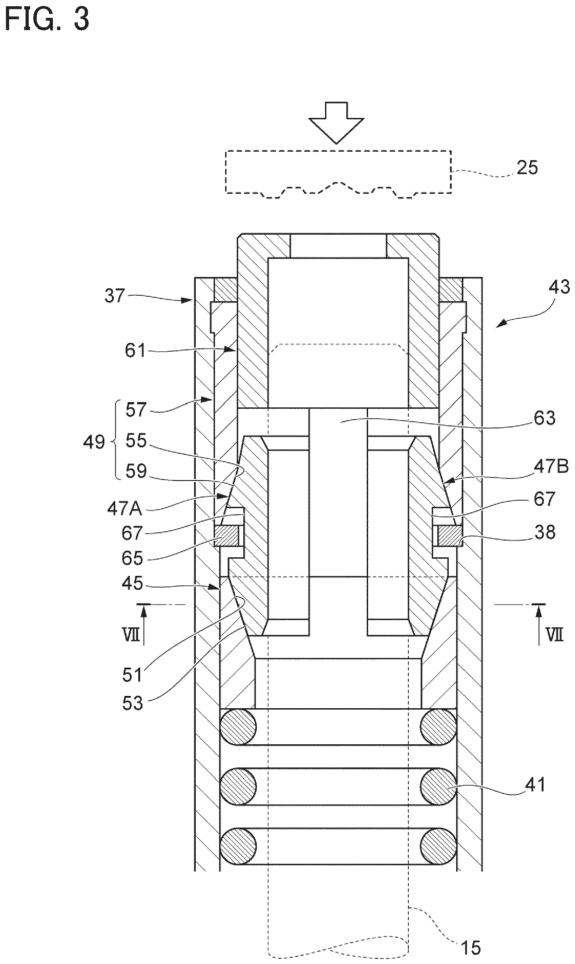

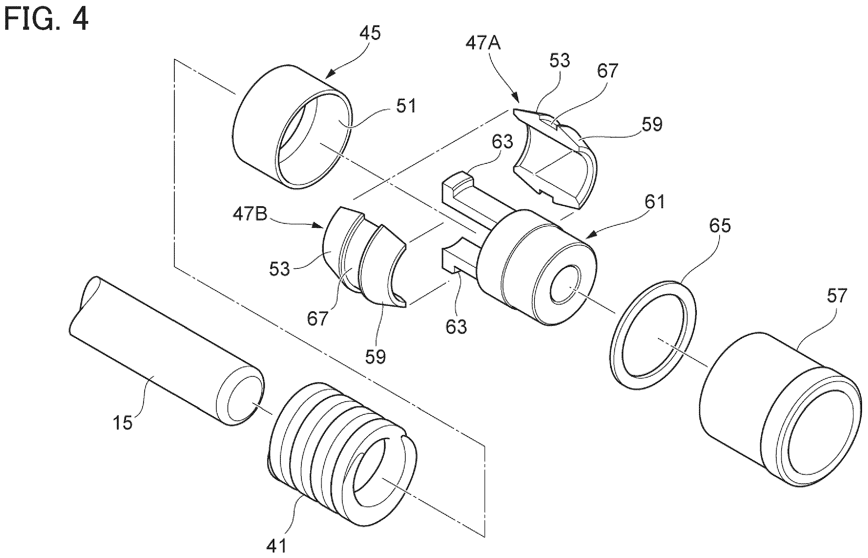

Fig. 3 is a partially enlarged cross-sectional view showing the chuck mechanism 43 housed in the sleeve 37. Fig. 4 is an exploded view showing the members constituting the chuck mechanism 43. In the following description, the same members or the same locations are denoted by the same reference numerals, so that descriptions thereof will be omitted or simplified.

The chuck mechanism 43 mainly includes the sleeve 37, the cylindrical slide member 45, a plurality of (two in the configuration) divided movable pieces 47A and 47B, the coil spring 41 described above, and a stopper unit 49 (see Fig. 3).

The cylindrical slide member 45 is housed in the sleeve 37 so as to be movable in an axial direction of the sleeve 37, and includes, on an inner peripheral surface thereof, a first tapered surface 51 whose diameter increases upward (forward in the insertion direction of the dental treatment tool 15). The divided movable pieces 47A and 47B each include, on an outer peripheral surface thereof, a second tapered surface 53 that comes into sliding contact with the first tapered surface 51.

The pair of divided movable pieces 47A and 47B are disposed so as to face each other in a diameter direction of the sleeve 37, and independently rotate in the sleeve 37 together with a pusher 61 which will be described later.

The stopper unit 49 includes a cylindrical fixing member 57 that includes a third tapered surface 55 (third tapered surface) on an inner peripheral surface thereof, and a fourth tapered surface 59 provided on each of the divided movable pieces 47A and 47B. These tapered surfaces each have a gradient in a direction of the axis Lc, thereby functioning as tapered fitting portions that convert movement of the divided movable pieces 47A and 47B in the direction of the axis Lc into movement of the divided movable pieces 47A and 47B in a radial direction. As will be described in detail later, the stopper unit 49 is at least partially fixed to the sleeve 37, and restricts forward axial movement of the divided movable pieces 47A and 47B in the insertion direction.

Furthermore, the pusher 61, which is moved by the push button 25 that is pressed down when the dental treatment tool 15 is to be detached from the chuck mechanism 43, is disposed above the divided movable pieces 47A and 47B. The pusher 61 includes a pair of push pieces 63 that extend toward the cylindrical slide member 45 through circumferential gaps between the divided movable pieces 47A and 47B.

An annular restriction ring 65 fixed to the sleeve 37 is disposed below the cylindrical fixing member 57 (at a rear end portion thereof in the insertion direction of the dental treatment tool 15). The restriction ring 65 is held between a stepped portion 38 formed on the inner peripheral surface of the sleeve 37 and a lower end of the cylindrical fixing member 57.

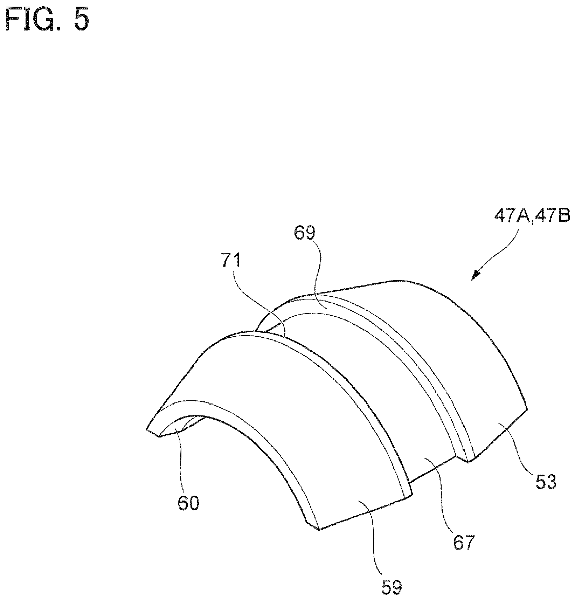

Fig. 5 is a perspective view of the divided movable pieces 47A and 47B.

The divided movable pieces 47A and 47B are disposed at different circumferential positions in the sleeve 37, and have the same shape. The divided movable pieces 47A and 47B each include the outer peripheral surface that comes into contact with the inner peripheral surface of the cylindrical slide member 45, and an inner peripheral surface that comes into contact with an outer peripheral surface of the dental treatment tool 15 inserted into the sleeve 37, and the divided movable pieces 47A and 47B are disposed such that the dental treatment tool 15 is held therebetween.

The second tapered surface 53 and the fourth tapered surface 59, which are gradually reduced in radius of an outer peripheral surface outward in the axial direction, are formed on one end side and the other end side of each of the divided movable pieces 47A and 47B in the axial direction. A recessed groove 67 along a circumferential direction is formed between the second tapered surface 53 and the fourth tapered surface 59. A groove wall 69 on a second tapered surface 53 side and a groove wall 71 on a fourth tapered surface 59 side are formed by the recessed groove 67. As shown in Fig. 3, the restriction ring 65 is inserted into the recessed groove 67. A cutout portion 60, which gradually increases in radius of an inner peripheral surface outward in the axial direction, is formed at at least one end portion of the inner peripheral surface of each of the divided movable pieces 47A and 47B.

Each of the divided movable pieces 47A and 47B shown here has a symmetrical shape in which a plane passing through an axial center and perpendicular to the axial direction is used as a symmetry plane. Since the divided movable piece has the symmetrical shape, it is not necessary to consider directionality of the divided movable piece when the chuck mechanism 43 is assembled, and assembling workability may be improved.

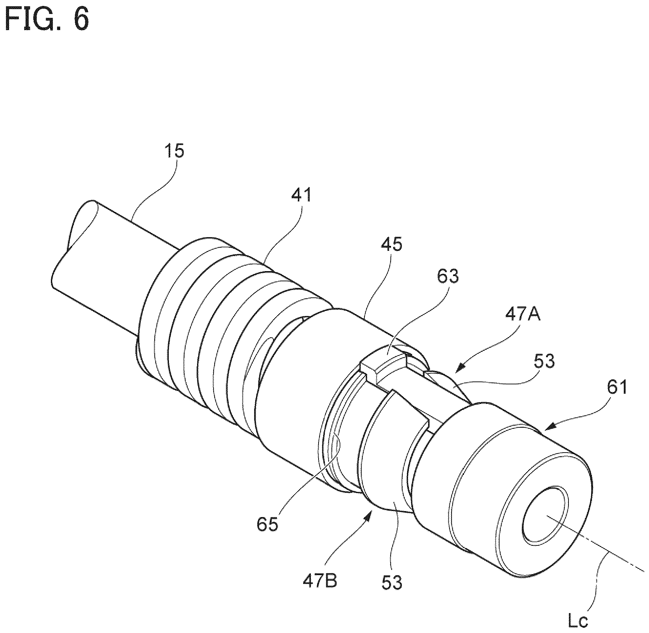

Fig. 6 is a perspective view showing a state after the members shown in Fig. 4 are assembled. In Fig. 6, the cylindrical fixing member 57 is not shown. In the pusher 61, a tip end of each of the push pieces 63 disposed between the pair of divided movable pieces 47A and 47B is in contact with the cylindrical slide member 45. By pressing the push button 25 down (see Fig. 3), the cylindrical slide member 45 is moved along the axis Lc against an elastic force of the coil spring 41.

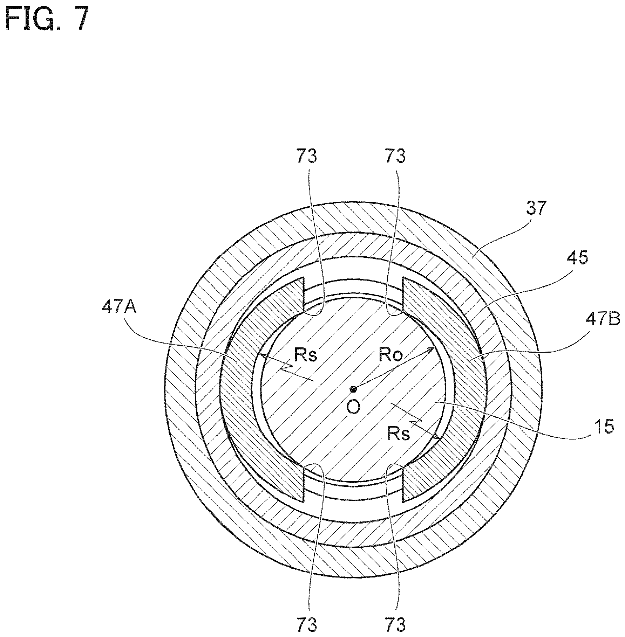

Fig. 7 is a cross-sectional view taken along a line VII-VII shown in Fig. 3.

A radius Rs of the inner peripheral surface of each of the divided movable pieces 47A and 47B is smaller than a radius R0 of the outer peripheral surface of the gripped dental treatment tool 15 (Rs < R0). In this case, when the divided movable pieces 47A and 47B grip the dental treatment tool 15, each of circumferential end portions 73 of the inner peripheral surface of each of the divided movable pieces 47A and 47B comes into line contact with the outer peripheral surface of the dental treatment tool 15. As a result, the circumferential end portions 73 are easy to elastically bite into the dental treatment tool 15, and reliable chucking of the dental treatment tool 15 may be realized.

### <Chucking Operation>

Next, a chucking operation of the dental treatment tool 15 performed by the chuck mechanism having the above configuration will be described.

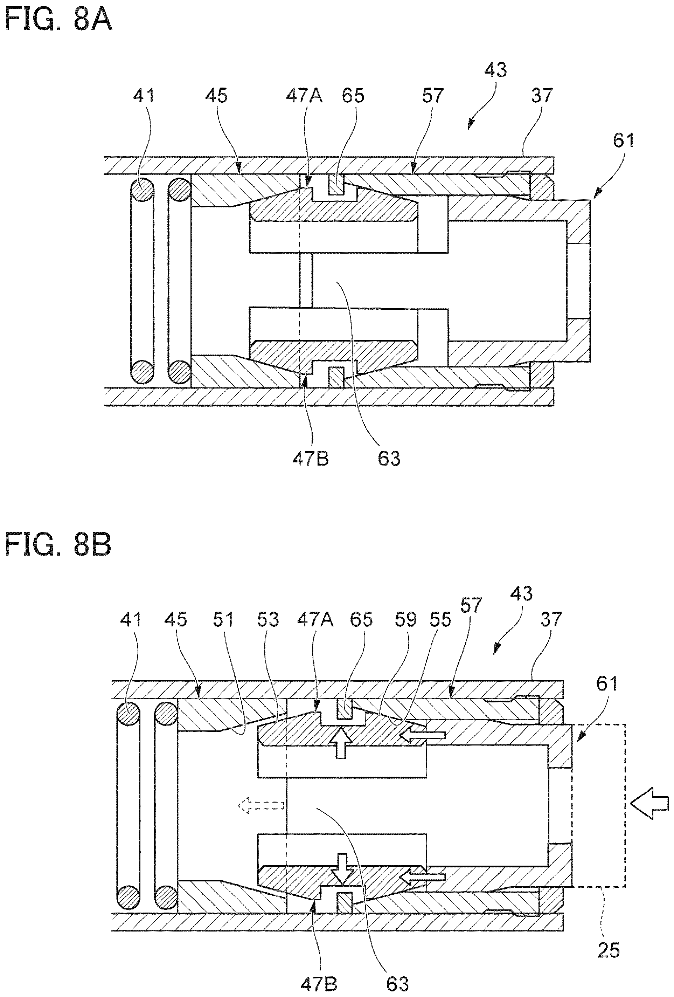

Figs. 8A to 8D are views stepwisely illustrating a process of performing the chucking operation by inserting a dental treatment tool into the chuck mechanism 43. Although illustration is simplified, rearward movement of the restriction ring 65 in the insertion direction is restricted by the stepped portion 38 provided on the inner peripheral surface of the sleeve 37 shown in Fig. 3.

### From a state shown in Fig. 8A, as shown in Fig. 8B, by pressing the push button 25 down, the pusher 61 is moved in a direction opposite to the insertion direction of the dental treatment tool 15. Then, the tip end of each of the push pieces 63 of the pusher 61 presses an end surface of the cylindrical slide member 45, so that the pusher 61 and the cylindrical slide member 45 are moved in the direction opposite to the insertion direction against a biasing force of the coil spring 41. As a result, the divided movable pieces 47A and 47B are separated from the cylindrical slide member 45.

When the push button 25 is further pressed down, an end portion of the pusher 61 in the direction opposite to the insertion direction comes into contact with the divided movable pieces 47A and 47B, so that the divided movable pieces 47A and 47B are moved in the direction opposite to the insertion direction. As a result, since the fourth tapered surface 59 slides on the third tapered surface 55, the divided movable pieces 47A and 47B are moved outward in the radial direction. An operation of pressing the push button 25 down is limited to a position where the groove wall 71 of each of the divided movable pieces 47A and 47B comes into contact with the restriction ring 65.

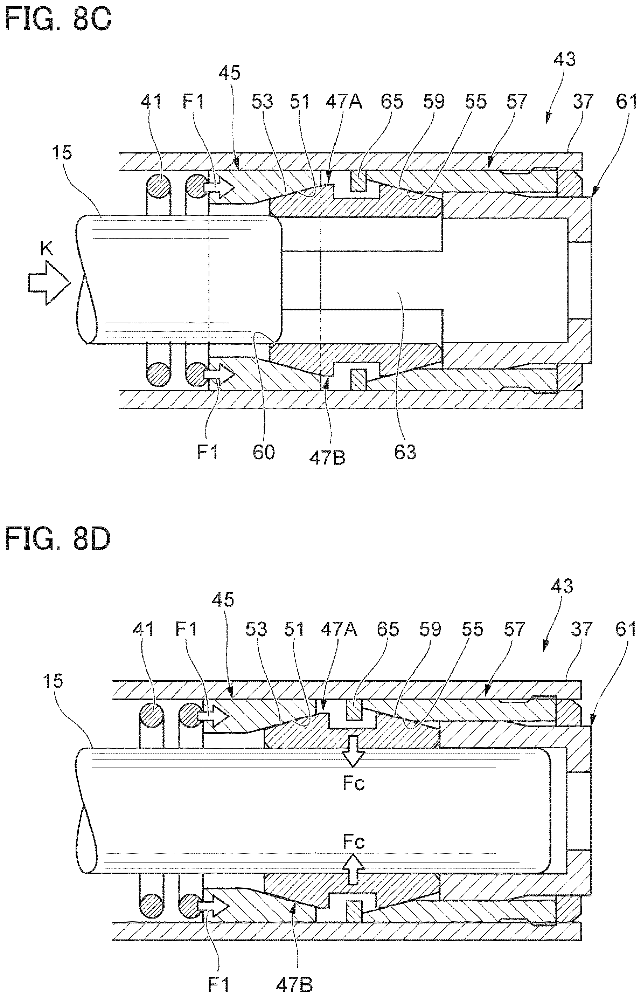

In this state, the dental treatment tool 15 is inserted into the sleeve 37 in a direction of an arrow K, and as shown in Fig. 8C, a tip end of the dental treatment tool 15 is inserted into the inner peripheral surfaces of the divided movable pieces 47A and 47B. At this time, the tip end of the dental treatment tool 15 comes into contact with the chamfered portion 60 formed at the end portion of the inner peripheral surface of each of the divided movable pieces 47A and 47B, and the dental treatment tool 15 is inserted while the divided movable pieces 47A and 47B are pushed so as to be moved outward. Radial positions of the divided movable pieces 47A and 47B are adjusted by sliding of the first tapered surface 51 and the second tapered surface 53 and sliding of the fourth tapered surface 59 on the third tapered surface 55 which are associated with an insertion operation of the dental treatment tool 15.

That is, when an outer peripheral surface of the tip end of the dental treatment tool 15 is inserted into inner peripheral sides of the divided movable pieces 47A and 47B, the divided movable pieces 47A and 47B are disposed at the radial positions corresponding to an outer diameter of the dental treatment tool 15. Since the divided movable pieces 47A and 47B are rotatably disposed in the sleeve 37, it becomes easy to match an outer peripheral surface of the dental treatment tool 15 and inner circumferential surfaces of the divided movable pieces 47A and 48B, and catching is less likely to occur when the dental treatment tool 15 is inserted.

In a case where the dental treatment tool 15 is inserted to a predetermined position in the sleeve 37, an operator release a pushing of the push button. As shown in Fig. 8D, by a biasing force F1 from the coil spring 41, the cylindrical slide member 45 is pushed and moved forward in the insertion direction of the dental treatment tool 15. Then, a wedge effect between the first tapered surface 51 and the second tapered surface 53, and a wedge effect between the third tapered surface 55 and the fourth tapered surface 59 are generated. By the wedge effect, clamping force Fc is generated and applied to the dental treatment tool 15, and the dental treatment tool 15 is chucked inside the sleeve 37 by the clamping force Fc.

### <Operation of Releasing Chucking>

Next, an operation of releasing chucking will be described.

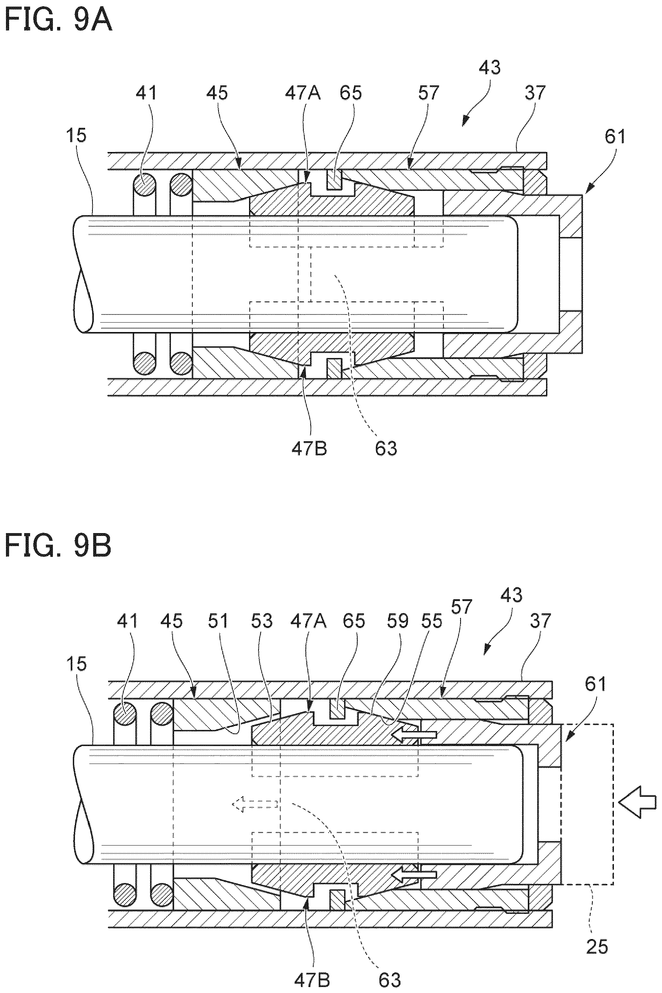

Figs. 9A to 9D are views stepwisely illustrating the operation of releasing the chucking of the dental treatment tool inserted into the chuck mechanism 43.

From a chucked state of the dental treatment tool 15 shown in Fig. 9A, as shown in Fig. 9B, the pusher 61 is moved in the direction opposite to the insertion direction of the dental treatment tool 15 by pressing the push button 25 down. Then, as described above, the pusher 61 and the cylindrical slide member 45 are moved in the direction opposite to the insertion direction against the biasing force of the coil spring 41, so that the divided movable pieces 47A and 47B are separated from the cylindrical slide member 45.

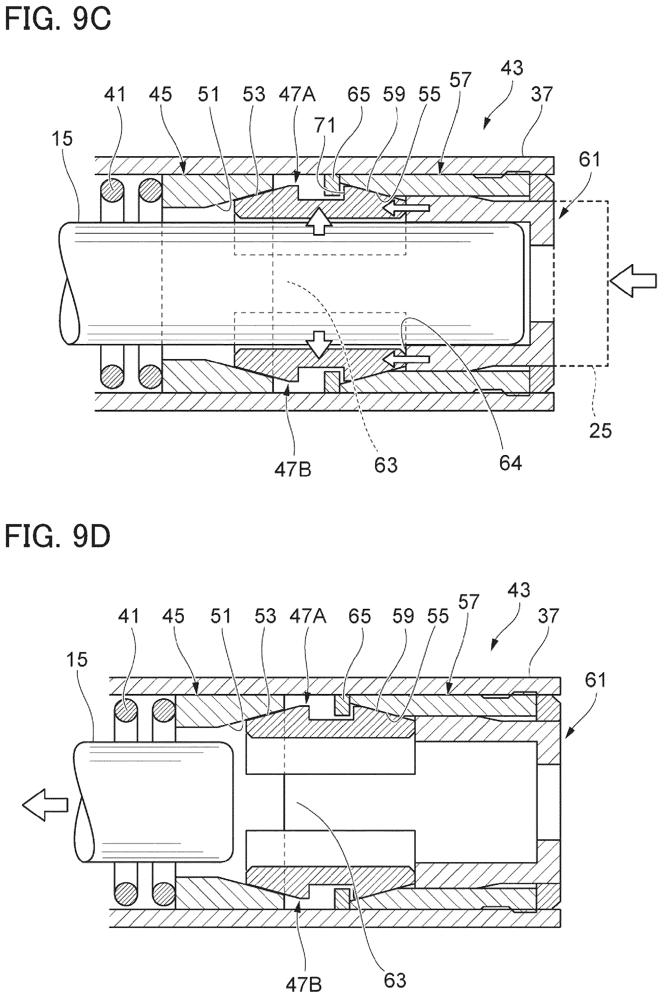

When the push button 25 is further pressed down, as shown in Fig. 9C, the divided movable pieces 47A and 47B are moved in the direction opposite to the insertion direction, so that the divided movable pieces 47A and 47B are moved outward in the radial direction by sliding of the fourth tapered surface 59 on the third tapered surface 55.

When the divided movable pieces 47A and 47B are moved outward in the radial direction as described above, the chucking of the dental treatment tool 15 is released and the dental treatment tool 15 may be extracted as shown in Fig. 9D.

That is, the outer peripheral surface of each of the divided movable pieces 47A and 47B and the inner peripheral surface of the cylindrical slide member 45 come into contact with each other via tapered fitting portions each having a gradient in the axial direction, and the divided movable pieces 47A and 47B are moved in the radial direction by relative movement between each of the divided movable pieces 47A and 47B and the cylindrical slide member 45 in the axial direction, so that fixation of the dental treatment tool 15 and release of the fixation are performed.

### <Application of External Force from Dental Treatment Tool>

When the dental handpiece is used, vibration and an axial force from the dental treatment tool propagate to the chuck mechanism. Even in such a case, the chuck mechanism 43 having the configuration may maintain reliable chucking without loosening the chucking of the dental treatment tool 15.

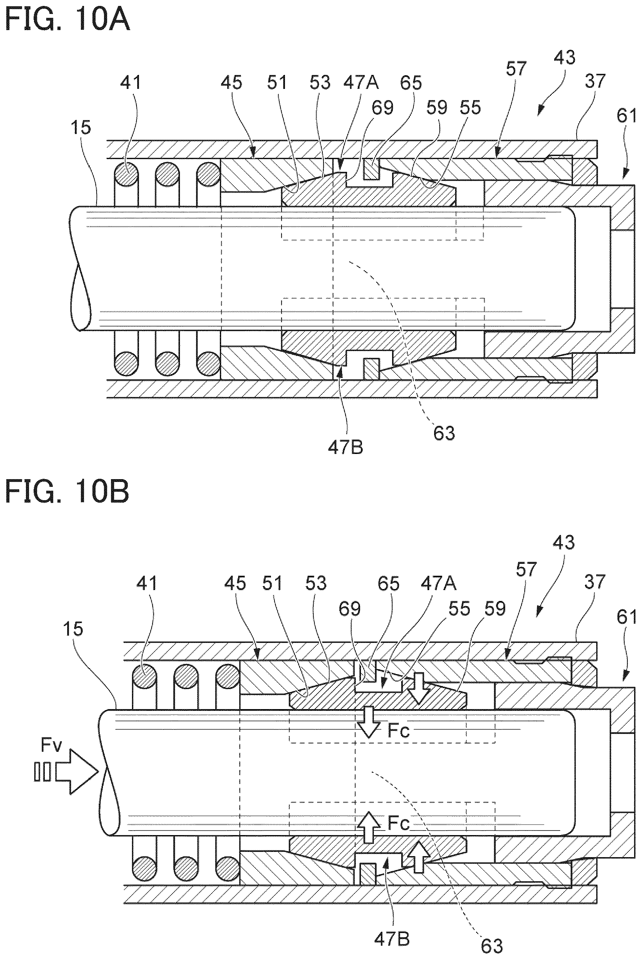

Figs. 10A to 10C are views stepwisely illustrating an operation of the chuck mechanism when an external force is applied to the chuck mechanism 43.

When an external force Fv is applied to the dental treatment tool 15 as shown in Fig. 10B from a chucked state of the dental treatment tool 15 shown in Fig. 10A, the dental treatment tool 15 is pushed into the sleeve 37 forward in the insertion direction, and a surface pressure between the third tapered surface 55 of the cylindrical fixing member 57 and the fourth tapered surface 59 of each of the divided movable pieces 47A and 47B increases. As a result, the clamping force Fc applied to the dental treatment tool 15 by each of the divided movable pieces 47A and 47B increases, and the dental treatment tool 15 is chucked more firmly. At this time, the cylindrical slide member 45 and each of the divided movable pieces 47A and 47B are moved together while being in contact with each other by the coil spring 41.

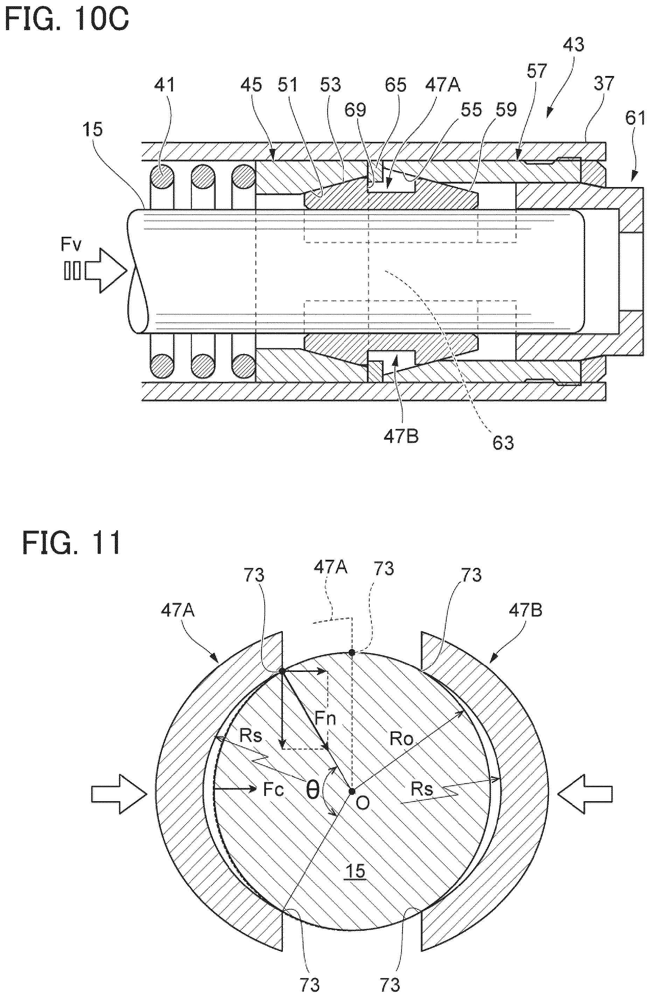

As shown in Fig. 10C, when the external force Fv further increases, the dental treatment tool 15 is moved forward in the insertion direction, and the groove wall 69 of each of the divided movable pieces 47A and 47B comes into contact with the restriction ring 65, so that further axial movement is restricted.

As described above, even when the vibration and the axial force act on the dental treatment tool 15, the chuck mechanism 43 always continues to chuck the dental treatment tool 15, and the chucking is not loosened.

Fig. 11 is a schematic cross-sectional view showing a state where the dental treatment tool 15 is held between the divided movable pieces 47A and 47B.

The clamping force Fc (see Figs. 8D and 10B) due to tapered fitting described above is converted into a normal force Fn for chucking the dental treatment tool 15 via the divided movable piece 47A. As a central angle θ formed by connecting the pair of circumferential end portions 73 of each of the divided movable pieces 47A and 47B with a center O of the dental treatment tool 15 increases, the normal force Fn increases. When a radius of the inner peripheral surface of each of the divided movable pieces 47A and 47B is set as Rs and a radius of the outer peripheral surface of the dental treatment tool 15 is set as R0, if Rs is greater than or equal to R0, the normal force Fn at the circumferential end portion 73 is almost zero, and a chucking force (biting) of the dental treatment tool 15 is reduced. Therefore, the radius Rs of the inner peripheral surface of each of the divided movable pieces 47A and 47B that grip the dental treatment tool 15 is preferably smaller than the radius R0 of the outer peripheral surface of the dental treatment tool 15.

When a circumferential length of each of the divided movable pieces 47A and 47B is extended to be close to a half of a perimeter of a circle (a position of the divided movable piece 47A indicated by a dotted line in Fig. 11), the normal force Fn that contributes to the chucking increases, but the divided movable pieces 47A and 47B approach each other and interfere with the push pieces 63 (see Fig. 4). If interference occurs, the clamping force Fc is a force to sandwich the push piece 63, and the dental treatment tool 15 may not be chucked. Since positions of the divided movable pieces 47A and 47B at the time of chucking tend to change greatly depending on a magnitude of the outer diameter of the dental treatment tool 15, a range of the outer diameter of the dental treatment tool 15 is limited.

Therefore, it is preferable that the divided movable pieces 47A and 47B are formed to have the circumferential length that allows a circumferential length of at least one sixth or more, preferably one fourth or more, and more preferably one third or more of an outer periphery of the dental treatment tool 15 to be exposed. That is, the central angle θ formed by connecting the pair of circumferential end portions 73 of each of the divided movable pieces 47A and 47B with a center O of the dental treatment tool 15 is preferably 165 degrees or less, more preferably 152.5 degrees or less, and further preferably 150 degrees or less. By setting each of the divided movable pieces 47A and 47B within the above range, it is possible to expand the range of the outer diameter of the dental treatment tool 15 that may be chucked.

Tapered fitting of each of the divided movable pieces 47A and 47B with the cylindrical slide member 45 and the cylindrical fixing member 57 needs to be set to a taper angle (an angle formed by an axis of the sleeve 37 and a tapered surface) that allows reliable chucking and smooth chucking release. In order to generate a sufficient chucking force while keeping a pressing-down stroke of the push button 25 at a very small amount of 1 mm or less from the viewpoint of operability, the taper angle is preferably 5 degrees or more, more preferably 7 degrees or more, and further preferably 10 degrees or more. For the same reason, the taper angle is preferably 30 degrees or less, more preferably 20 degrees or less, and further preferably 15 degrees or less.

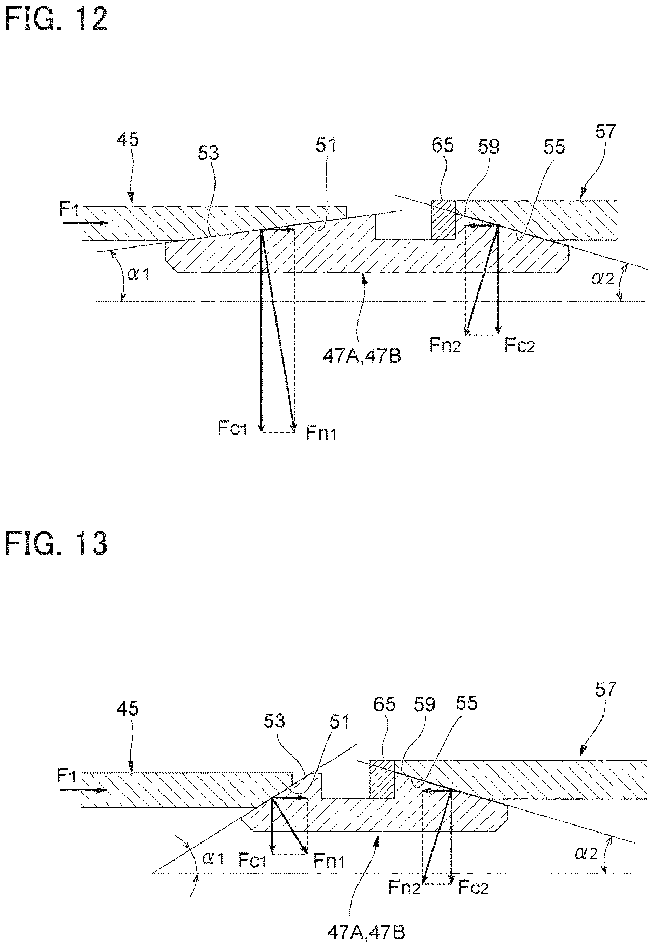

Figs. 12 and 13 are views each illustrating a case where a taper angle of the first tapered surface 51 and the second tapered surface 53 is different from a taper angle of the third tapered surface 55 and the fourth tapered surface 59.

Each of the divided movable pieces 47A and 47B has the symmetrical shape in which the plane passing through the axial center and perpendicular to the axial direction is used as the symmetry plane, and a taper angle α1 of the first tapered surface 51 and the second tapered surface 53 is equal to a taper angle α2 of the third tapered surface 55 and the fourth tapered surface 59, but the taper angles are not limited thereto. The taper angles may be different from each other on one side and the other side, in the axial direction, of the plane perpendicular to the axial direction.

As shown in Fig. 12, when the taper angle α1 is smaller than the taper angle α2, an inclination of the tapered surface with respect to the axial direction is reduced, and the biasing force F1 from the coil spring 41 (Fig. 3) may be converted into a larger normal force Fn1. Since a clamping force Fc1 is a component of a force of Fn1 in a direction perpendicular to a rotation axis, the clamping force Fc1 may also be increased.

As shown in Fig. 13, when the taper angle α1 is larger than the taper angle α2, an inclination of the tapered surface with respect to the axial direction increases, and the normal force Fn1 is reduced. Therefore, a frictional force generated on the tapered surface may be reduced, and occurrence of biting may be prevented.

Although the divided movable pieces 47A and 47B have a configuration in which dividing into two parts in the circumferential direction is performed, the number of divisions is not limited thereto. For example, dividing into three parts or four parts in the circumferential direction may be performed. However, since a chucking force generated on one divided movable piece due to tapered fitting is reduced as the number of divisions increases, it is preferable to reduce the number of divisions when a radial force due to the tapered fitting is small.

According to the chuck mechanism 43 described above, the divided movable pieces 47A and 47B are formed into a two-piece structure and each provided with tapered surfaces, so that a wedge effect may be produced, and a small force in a direction of the rotation axis generated by the coil spring 41 may be converted into a large force in the radial direction. By providing the tapered surfaces at both ends of each of the divided movable pieces 47A and 47B, even when a force in the insertion direction or the direction opposite to the insertion direction (extraction direction) is applied to the dental treatment tool 15, the outer peripheral surface of the dental treatment tool 15 are not separated from the divided movable pieces 47A and 47B, and the dental treatment tool 15 does not drop out carelessly or is not excessively inserted.

Each of the divided movable pieces 47A and 47B is provided with the tapered surfaces at two locations, so that an effect of converting a direction of a force by a taper may be doubled, and a sufficient radial force may be obtained even when the taper angle increases. Biting that causes attachment and detachment failures of the dental treatment tool 15 is less likely to occur. Furthermore, since the dental treatment tool 15 is chucked while the outer peripheral surface of the dental treatment tool 15 is in line contact with the inner peripheral surface of each of the divided movable pieces 47A and 47B, a region that contributes to the chucking is widened. As a result, it is possible to obtain a configuration that is resistant to wear and has high durability. In the chuck mechanism 43, a member requiring an elastic force and a member requiring rigidity are separated from each other, so that a material having characteristics required for each member may be freely selected, and a degree of freedom in design may be improved.

Sliding members (the divided movable pieces 47A and 47B, the cylindrical slide member 45, and the cylindrical fixing member 57) that each slide on the tapered surface are formed separately from the coil spring 41 serving as the elastic biasing member. Therefore, as compared with a case where the sliding members also function as an elastic biasing member, the sliding members may be formed of a material having a low spring property and higher hardness. As a result, it is possible to further improve wear resistance of the divided movable pieces 47A and 47B, the cylindrical slide member 45, and the cylindrical fixing member 57.

In the tapered fitting described above, each of the divided movable pieces 47A and 47B, the cylindrical slide member 45, and the cylindrical fixing member 57 is provided with the tapered surface, but only one of the tapered surfaces that are fitted together may be formed as the tapered surface. When such an one-side taper is used, a structure of the chuck mechanism 43 may be simplified.

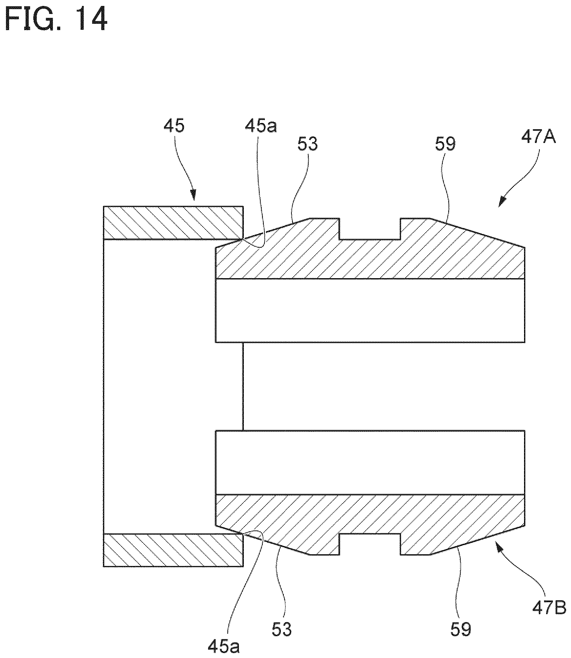

Fig. 14 is a schematic cross-sectional view showing the divided movable pieces 47A and 47B that each include the second tapered surface 53 on the outer peripheral surface, and the cylindrical slide member 45 that includes no first tapered surface on the inner peripheral surface.

As shown in Fig. 14, the cylindrical slide member 45 may not be provided with the first tapered surface, and an end portion 45a of the inner peripheral surface of the cylindrical slide member 45 may slide on the second tapered surface 53. Similarly, the third tapered surface 55 may not be provided on the inner peripheral surface of the cylindrical fixing member 57 shown in Fig. 3, and an end portion of the inner peripheral surface of the cylindrical fixing member 57 may slide on the fourth tapered surface 59 of each of the divided movable pieces 47A and 47B. Even in such a case, the chucking operation described above may be performed.

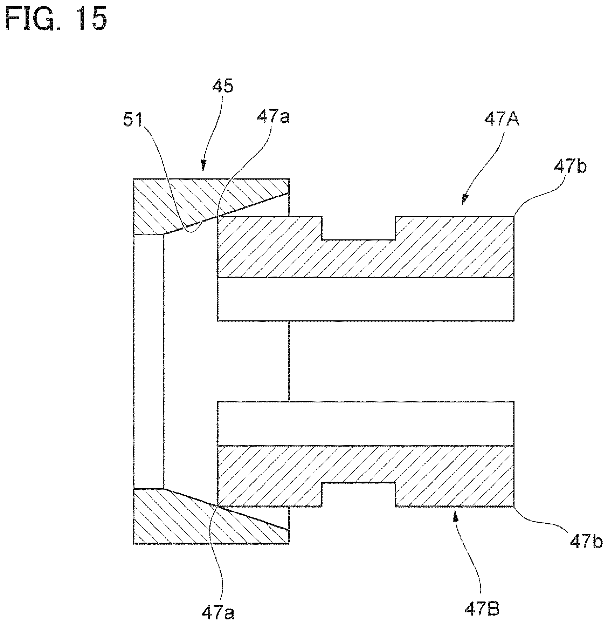

Fig. 15 is a schematic cross-sectional view showing the divided movable pieces 47A and 47B that each include no second tapered surface 53 on the outer peripheral surface, and the cylindrical slide member 45 that includes the first tapered surface on the inner peripheral surface.

As shown in Fig. 15, each of the divided movable pieces 47A and 47B may not be provided with the second tapered surface, and one end portion 47a of the outer peripheral surface of each of the divided movable pieces 47A and 47B may slide on the first tapered surface 51 of the cylindrical slide member 45. Similarly, each of the divided movable pieces 47A and 47B may not be provided with the fourth tapered surface, and the other end portion 47b of the outer peripheral surface of each of the divided movable pieces 47A and 47B may slide on the third tapered surface 55 on the inner peripheral surface of the cylindrical fixing member 57 shown in Fig. 3. Even in such a case, the chucking operation described above may be performed.

Furthermore, the above configurations may also be appropriately combined, for example, by not providing the tapered surface on one side, in the axial direction, of each of the divided movable pieces 47A and 47B, and providing the tapered surface only on the other side in the axial direction. The above configurations may be similarly applied to each modification to be described below.

### <Other Configuration Examples of Chuck Mechanism>

### (First Modification)

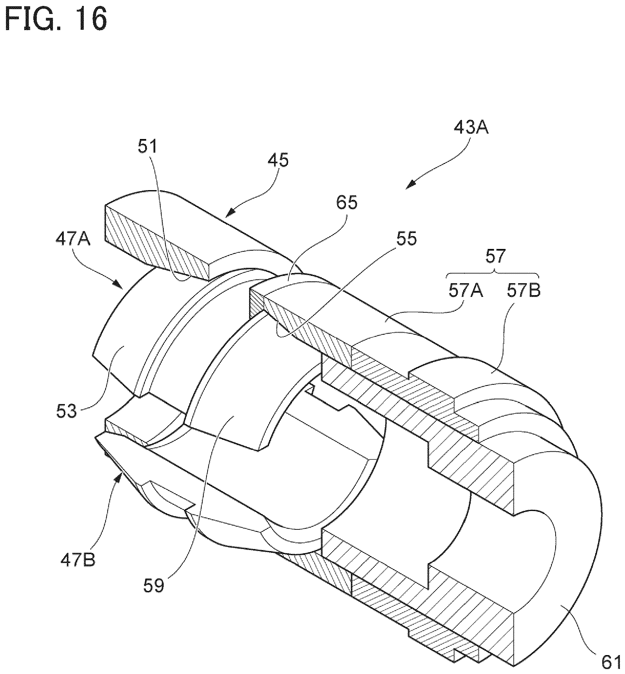

Fig. 16 is a partial cross-sectional view of a chuck mechanism 43A according to a first modification including the cylindrical fixing member 57 that is divided into two parts. The cylindrical fixing member 57 of the chuck mechanism 43A is divided, along the axial direction, into a tapered cylindrical member 57A in a region including the third tapered surface 55, and a remaining cylindrical member 57B in a region excluding the tapered cylindrical member 57A.

The third tapered surface 55 is formed on the inner peripheral surface of the cylindrical fixing member 57, and the third tapered surface 55 performs tapered fitting with the fourth tapered surface 59 and is thus required to have high machining accuracy and high wear resistance. However, for the region other than the third tapered surface 55, such conditions are often not required. Therefore, it is preferable to, as shown in Fig. 16, divide the cylindrical fixing member 57 into the region including the third tapered surface 55 for which particularly high machining accuracy and wear resistance are required, and the other region. In this case, for example, the tapered cylindrical member 57A may be formed by machining, with high accuracy, a material having high wear resistance, and the remaining cylindrical member 57B may be formed using a material that is relatively low in cost and good in machinability. That is, the cylindrical fixing member 57 may be formed with little waste so that necessary performance may be sufficiently exhibited while reducing material cost. Since an axial length of the tapered cylindrical member 57A is shortened, machinability thereof is improved.

### (Second Modification)

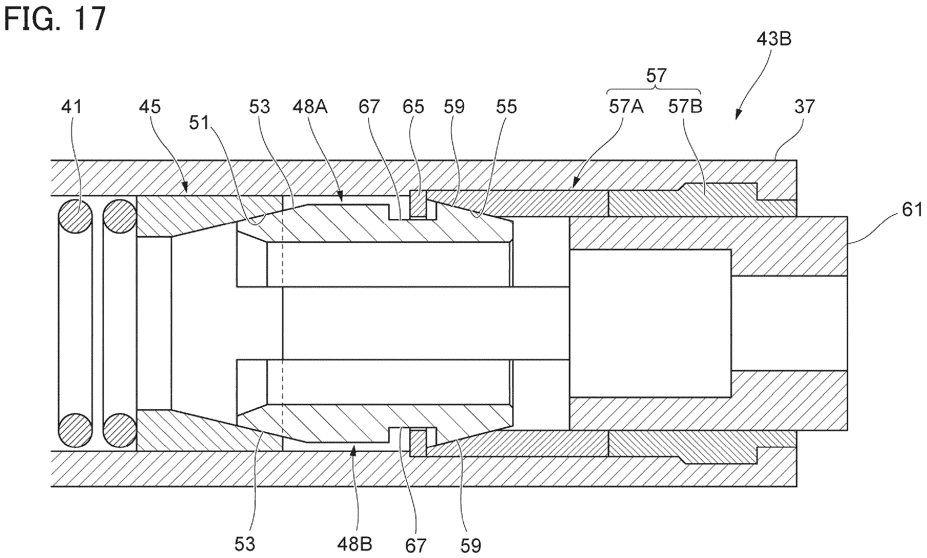

Fig. 17 is a cross-sectional view showing a chuck mechanism 43B according to a second modification that includes divided movable pieces 48A and 48B whose axial length is extended.

A length from the recessed groove 67 to the second tapered surface 53 of each of the divided movable pieces 48A and 48B of the chuck mechanism 43B is extended. According to the configuration, when an inclination of the second tapered surface 53 is set to be small, a space for forming the tapered surface is easily ensured. As a result, it is possible to obtain the configuration in which a contact area between the first tapered surface 51 and the second tapered surface 53 may be increased so as to reduce a surface pressure and reduce wear. Similarly, a contact area may be increased by extending a length from the recessed groove 67 to the fourth tapered surface 59.

### (Third Modification)

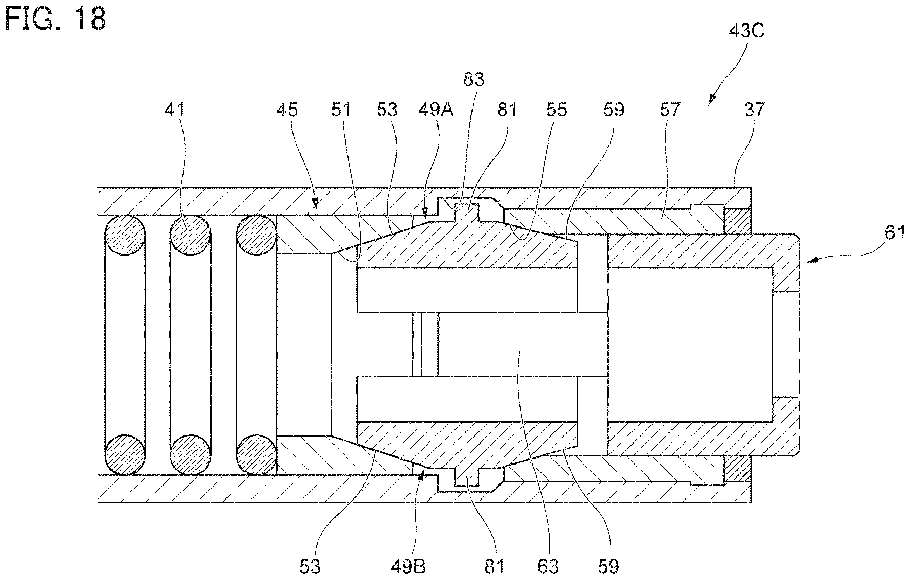

Fig. 18 is a cross-sectional view showing a chuck mechanism 43C according to a third modification in which each of protruding portions is formed integrally with a respective one of divided movable pieces 49A and 49B.

Each of the divided movable pieces 49A and 49B of the chuck mechanism 43C is formed, between the second tapered surface 53 and the fourth tapered surface 59, with a protruding portion 81 which is formed along the circumferential direction and protrudes outward in the radial direction. The protruding portion 81 may come into contact with one of end portions of the cylindrical slide member 45 and the cylindrical fixing member 57 facing each other. The sleeve 37 is formed with an annular groove portion 83 that prevents interference when the divided movable pieces 49A and 49B each including the protruding portion 81 are moved outward in the radial direction.

According to the configuration, a structure may be simplified by omitting the restriction ring 65 (Fig. 3) described above, and movement of the divided movable pieces 49A and 49B in the axial direction may be restricted.

### (Fourth Modification)

The configuration of each of chuck mechanisms described above may also be further simplified.

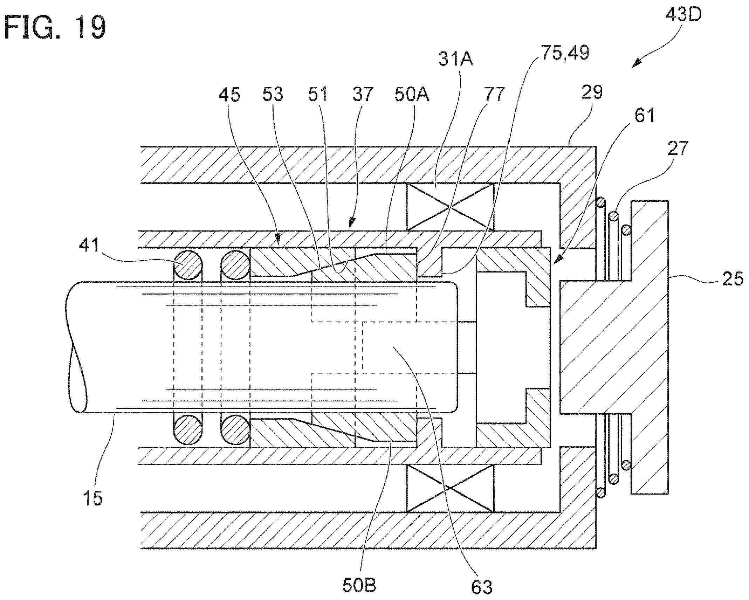

Fig. 19 is a schematic cross-sectional view showing a simplified configuration of a chuck mechanism 43D according to a fourth modification.

In the chuck mechanism 43D, axial movement of divided movable pieces 50A and 50B each including the second tapered surface 53 is restricted by a protruding portion 75 that protrudes inward from the inner peripheral surface of the sleeve 37 in the radial direction. Other configurations may be the same as those of the chuck mechanisms 43, 43A, and 43B described above.

Each of the divided movable pieces 50A and 50B includes, on one end side in the axial direction, a tapered surface (second tapered surface 53) that is in sliding contact with the first tapered surface 51 of the cylindrical slide member 45, and includes, on the other end side in the axial direction, an end surface 77 that is in contact with the protruding portion 75. That is, the fourth tapered surface 59 described above is not provided.

The protruding portion 75 may be formed on the inner peripheral surface of the sleeve 37 continuously in the circumferential direction, or may be a plurality of protruding portions obtained by dividing in the circumferential direction. The protruding portion 75 only needs to be in contact with the end surface 77 of each of the divided movable pieces 50A and 50B, and functions as the stopper unit 49 that restricts the axial movement of the divided movable pieces 50A and 50B.

According to the chuck mechanism 43D, reliable chucking of the dental treatment tool 15 may be performed by tapered fitting of the first tapered surface 51 with the second tapered surface 53. Moreover, since a member that requires flexible elasticity such as the coil spring 41, and members that perform tapered fitting with each other and each have excellent wear resistance and high rigidity are separately formed, characteristics required for each member may be easily selected.

### (Fifth Modification)

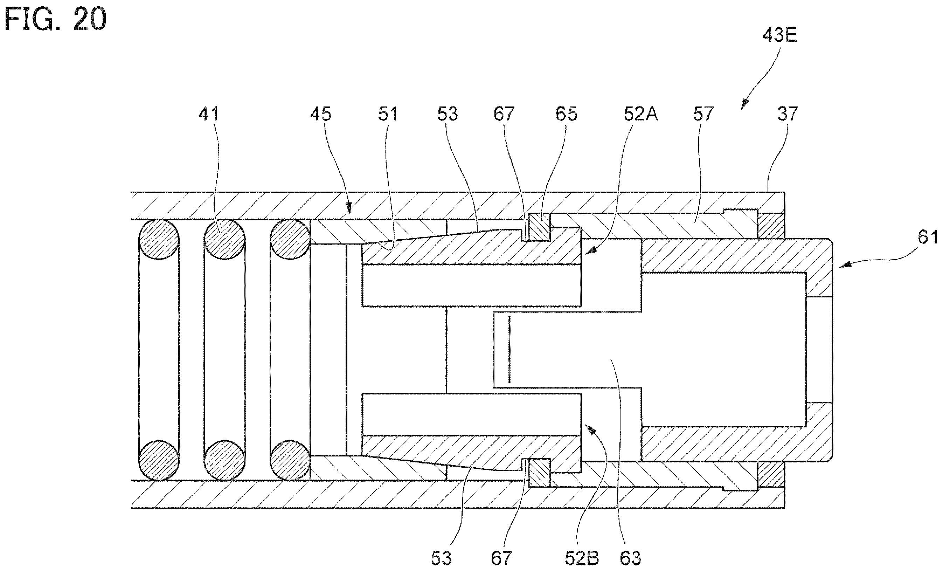

Fig. 20 is a schematic cross-sectional view showing another simplified configuration of a chuck mechanism 43E according to a fifth modification.

The chuck mechanism 43E includes divided movable pieces 52A and 52B that are each provided with a tapered surface only on one side in the axial direction, and axial movement of the divided movable pieces 52A and 52B is restricted by the restriction ring 65. An end portion of the cylindrical fixing member 57 on a restriction ring 65 side does not include a tapered surface (third tapered surface 55), and an end surface of the cylindrical fixing member 57 abuts against the restriction ring 65. Other configurations are the same as those of the chuck mechanism 43 shown in Fig. 3.

According to the configuration, similarly to the chuck mechanism 43D according to the fourth modification, the reliable chucking may be performed by the tapered fitting of the first tapered surface 51 with the second tapered surface 53, and machining of the sleeve 37 may be simplified.

The present disclosure is not limited to the above embodiments, and modifications, improvements, and the like are possible as appropriate. In addition, a material, a shape, a dimension, a numerical value, a form, the number, a disposition position, and the like of each of components in the above embodiments are not limited as long as the present disclosure may be achieved. The scope of the present invention being defined by the wording of the claims.

## Claims

1. A dental handpiece (100) configured to rotatably drive a dental treatment tool (15) mounted in a chuck mechanism, the dental hand piece comprising:
the chuck mechanism in which the dental treatment tool is mounted so as to be attachable and detachable,
wherein the chuck mechanism includes:
a hollow cylindrical sleeve (37) into which the dental treatment tool (15) is inserted so as to be rotatably supported;
a cylindrical slide member (45) housed in the sleeve so as to be movable in an axial direction of the sleeve;
a plurality of divided movable pieces (47A, 47B) disposed in the sleeve at different circumferential positions to each other, each of the plurality of divided movable pieces including an outer peripheral surface that comes into contact with an inner peripheral surface of the cylindrical slide member, and an inner peripheral surface that comes into contact with an outer peripheral surface of the dental treatment tool inserted into the sleeve, and the dental treatment tool being held between the plurality of divided movable pieces;
an elastic biasing member (41) configured to bias the cylindrical slide member forward in an insertion direction of the dental treatment tool; and
a stopper unit (49) configured to restrict forward axial movement of the plurality of divided movable pieces in the insertion direction, at least a part of the stopper unit being partially fixed to the sleeve, and
wherein the outer peripheral surface of each of the plurality of divided movable pieces (47A, 47B) and the inner peripheral surface of the cylindrical slide member (45) contact with each other via a tapered fitting portion (51, 53) having a gradient in the axial direction, the tapered fitting portion being provided on at least one of the outer peripheral surface of the plurality of divided movable pieces (47A, 47B) and the inner peripheral surface of the cylindrical slide member (45),
wherein the divided movable pieces (47A, 47B) are moved in a radial direction by relative movement between each of the divided movable pieces (47A, 47B) and the cylindrical slide member (45) in the axial direction, so that fixation of the dental treatment tool (15) and release of the fixation are performed, and
**characterized in that**
the plurality of divided movable pieces (47A, 47B) are disposed in the sleeve (37) so as to be rotatable with respect to the sleeve about an axis of the sleeve.

2. The dental handpiece according to claim 1,
wherein a pair of the divided movable pieces (47A, 47B) are disposed so as to face each other in a diameter direction of the sleeve (37).

3. The dental handpiece according to claim 1,
wherein the cylindrical slide member (45) includes, on the inner peripheral surface of the cylindrical slide member, a first tapered surface (51) whose diameter increases forward in the insertion direction, and
wherein each of the plurality of divided movable pieces (47A, 47B) includes, on the outer peripheral surface of the divided movable piece, a second tapered surface (53) that is configured to come into sliding contact with the first tapered surface.

4. The dental handpiece according to claim 3,
wherein the stopper unit (49) includes:
a cylindrical fixing member (57) that is disposed at a more forward position than the plurality of divided movable pieces (47A, 47B) in the insertion direction, and that includes, on an inner peripheral surface of the cylindrical fixing member, a third tapered surface (55) whose diameter is reduced forward in the insertion direction; and
a fourth tapered surface (59) that is provided on the outer peripheral surface of each of the plurality of divided movable pieces (47A, 47B) and that is configured to come into sliding contact with the third tapered surface.

5. The dental handpiece according to claim 4,
wherein the cylindrical fixing member (57) is divided, in the axial direction, into a tapered cylindrical member in a region including the third tapered surface, and a remaining cylindrical member in a region excluding the tapered cylindrical member.

6. The dental handpiece according to any one of claims 1 to 5,
wherein each of the divided movable pieces (47A, 47B) is provided, on the outer peripheral surface forward of the tapered fitting portion in the insertion direction, with a protruding portion (81) that protrudes outward in the radial direction, and
wherein the protruding portion is contactable with a forward end portion of the cylindrical slide member (45) in the insertion direction.

7. The dental handpiece according to claim 4 or 5, further comprising:
an annular restriction ring (65) that is disposed at a rear end portion of the cylindrical fixing member (57) in the insertion direction and that is fixed to the sleeve (37),
wherein in each of the divided movable pieces (47A, 47B), a recessed groove (67) is formed along a circumferential direction on an outer diameter surface between the second tapered surface (53) and the fourth tapered surface (55), and
wherein the restriction ring has a radial thickness such that an inner peripheral surface of the restriction ring is inserted into the recessed groove, and a forward groove wall or a rear groove wall of the recessed groove in the insertion direction comes into contact with the restriction ring by movement of the divided movable piece in the axial direction.

8. The dental handpiece according to claim 4 or 5,
wherein the divided movable pieces (47A, 47B), the cylindrical slide member (45), and the cylindrical fixing member (57) are each made of a material which is higher in hardness than the elastic biasing member (41).

9. The dental handpiece according to claim 4 or 5,
wherein in an axial cross section of the sleeve, a taper angle formed by an axis of the sleeve and at least one of the second tapered surface (53) and the fourth tapered surface (55) of each of the divided movable pieces (47A, 47B) is 5 degrees or more and 30 degrees or less.

10. The dental handpiece according to any one of claims 1 to 5, further comprising:
a pusher (61) that is disposed forward of the divided movable pieces (47A, 47B) in the insertion direction and that is pressed down in a case where the dental treatment tool (15) is to be detached from the chuck mechanism,
wherein the pusher includes a push piece (63) that extends toward the cylindrical slide member (45) through a circumferential gap between the plurality of divided movable pieces (47A, 47B), and
wherein in a case where the pusher (61) is pressed down rearward in the insertion direction of the dental treatment tool (15), the push piece (63) pushes back the cylindrical slide member (45) rearward in the insertion direction, so that chucking of the dental treatment tool by the divided movable pieces (47A, 47B) is released.

11. The dental handpiece according to any one of claims 1 to 5,
wherein a radius of the inner peripheral surface of each of the divided movable pieces (47A, 47B) is smaller than a radius of the outer peripheral surface of the dental treatment tool (15).

12. The dental handpiece according to any one of claims 1 to 5,
wherein the elastic biasing member includes a coil spring (41).

13. The dental handpiece according to any one of claims 1 to 5,
wherein each of the divided movable pieces (47A, 47B) has a symmetrical shape in which a plane passing through an axial center and perpendicular to the axial direction is used as a symmetry plane.

## Patentansprüche

1. Zahnärztliches Handstück (100), das konfiguriert ist, um ein zahnärztliches Behandlungswerkzeug (15), das an einem Spannfuttermechanismus befestigt ist, drehbar anzutreiben, wobei das zahnärztliche Handstück umfasst:
den Spannfuttermechanismus, in dem das zahnärztliche Behandlungswerkzeug so befestigt ist, dass es anbringbar und lösbar ist,
wobei der Spannfuttermechanismus enthält:
eine hohle zylindrische Muffe (37), in der das zahnärztliche Behandlungswerkzeug (15) eingesetzt ist, um so drehbar gelagert zu sein;
ein zylindrisches Seitenelement (45), das in der Muffe so untergebracht ist, dass es in einer axialen Richtung der Muffe beweglich ist;
mehrere geteilte bewegliche Stücke (47A, 47B), die in der Muffe an verschiedenen Umfangspositionen zueinander angeordnet sind, wobei jede der mehreren geteilten beweglichen Stücke eine äußere Umfangsoberfläche, die in einen Kontakt mit einer inneren Umfangsoberfläche des zylindrischen Seitenelements kommt, und eine innere Umfangsoberfläche, die in einen Kontakt mit einer äußeren Umfangsoberfläche des zahnärztlichen Behandlungswerkzeugs, das in der Muffe eingesetzt ist, kommt, enthält, und wobei das zahnärztliche Behandlungswerkzeug zwischen den mehreren geteilten beweglichen Stücken gehalten wird;
ein elastisches Vorspannelement (41), das konfiguriert ist, um das zylindrische Seitenelement vorwärts in eine Einsetzrichtung des Behandlungswerkzeugs vorzuspannen; und
eine Stoppereinheit (49), die konfiguriert ist, um die axiale Vorwärtsbewegung der mehreren geteilten beweglichen Stücke in die Einsetzrichtung zu beschränken, wobei mindestens ein Teil der Stoppereinheit teilweise an der Muffe befestigt ist, und
wobei sich die äußere Umfangsoberfläche von jeder der mehreren geteilten beweglichen Stücke (47A, 47B) und die innere Umfangsoberfläche des zylindrischen Seitenelements (45) gegenseitig in Kontakt miteinander bringen über einen konischen, passenden Abschnitt (51, 53), der in der axialen Richtung einen Gradienten aufweist, wobei der konische, passende Abschnitt entweder auf der äußeren Umfangsoberfläche der mehreren geteilten beweglichen Stücke (47A, 47B) oder auf der inneren Umfangsoberfläche des zylindrischen Seitenelements (45) bereitgestellt ist,
wobei die geteilten beweglichen Stücke (47A, 47B) in eine radiale Richtung durch eine relative Bewegung zwischen jedem der geteilten beweglichen Stücke (47A, 47B) und die zylindrischen Seitenelements (45) in die axiale Richtung so bewegt werden, dass die Befestigung des zahnärztlichen Behandlungswerkzeugs (15) und die Lösung der Befestigung durchgeführt werden, und
**dadurch gekennzeichnet, dass**
die mehreren geteilten beweglichen Stücke (47A, 47B) in der Muffe (37) so angeordnet sind, um in Bezug auf die Muffe um eine Achse der Muffe gedreht werden zu können.

2. Zahnärztliches Handstück nach Anspruch 1,
wobei ein Paar der geteilten beweglichen Stücke (47A, 47B) so angeordnet ist, um sich in einer Durchmesserrichtung der Muffe (37) zueinander gegenüberzuliegen.

3. Zahnärztliches Handstück nach Anspruch 1,
wobei das zylindrische Seitenelement (45) auf der inneren Umfangsoberfläche des zylindrischen Seitenelements eine erste konische Oberfläche (51) enthält, deren Durchmesser nach vorne in der Einsetzrichtung ansteigt, und
wobei jeder der mehreren geteilten beweglichen Stücke (47A, 47B) auf der äußeren Umfangsoberfläche des geteilten beweglichen Stücks eine zweite konische Oberfläche (53) enthält, die konfiguriert ist, um in einen Gleitkontakt mit der ersten konischen Oberfläche zu kommen.

4. Zahnärztliches Handstück nach Anspruch 3,
wobei die Stoppereinheit (49) enthält:
ein zylindrisches Befestigungselement (57), das in einer Position angeordnet ist, die sich weiter vorne befindet als die mehreren geteilten beweglichen Stücke (47A, 47B) in der Einsetzrichtung, und das auf einer inneren Umfangsoberfläche des zylindrischen Befestigungselements eine dritte konische Oberfläche (55) enthält, deren Durchmesser nach vorne in der Einsetzrichtung abnimmt; und
eine vierte konische Oberfläche (59), die auf der äußeren Umfangsoberfläche von jeder der mehreren geteilten beweglichen Stücke (47A, 47B) bereitgestellt ist, und die konfiguriert ist, um in einen Gleitkontakt mit der dritten konischen Oberfläche zu kommen.

5. Zahnärztliches Handstück nach Anspruch 4,
wobei das zylindrische Befestigungselement (57) in der axialen Richtung in ein konisches zylindrisches Element in einem Bereich, der die dritte konische Oberfläche enthält, und in ein verbleibendes zylindrisches Element in einem Bereich, der das konische zylindrische Element ausschließt, geteilt ist.

6. Zahnärztliches Handstück nach einem der Ansprüche 1 bis 5,
wobei jedes der geteilten beweglichen Stücke (47A, 47B) auf der äußeren Umfangsoberfläche vor dem konischen, passenden Abschnitt in der Einsetzrichtung mit einem hervorstehenden Abschnitt (81) versehen ist, der nach außen in der radialen Richtung hervorsteht, und
wobei der hervorstehende Abschnitt mit einem vorderen Endabschnitt des zylindrischen Seitenelements (45) in der Einsetzrichtung in Kontakt gebracht werden kann.

7. Zahnärztliches Handstück nach Anspruch 4 oder 5, das ferner umfasst:
einen ringförmigen Drosselring (65), der auf dem hinteren Endabschnitt des zylindrischen Befestigungselements (57) in der Einsetzrichtung angeordnet ist und der in der Muffe (37) befestigt ist,
wobei in jedem der geteilten beweglichen Stücke (47A, 47B) eine vertiefte Nut (67) entlang einer Umfangsrichtung auf einer äußeren Durchmesseroberfläche zwischen der zweiten konischen Oberfläche (53) und der vierten konischen Oberfläche (55) gebildet ist, und
wobei der Drosselring eine radiale Dicke derart aufweist, dass eine innere Umfangsoberfläche des Drosselrings in der vertieften Nut eingesetzt ist, und wobei eine vordere Nutwand oder eine hintere Nutwand der vertieften Nut in der Einsetzrichtung in einen Kontakt mit dem Drosselring durch eine Bewegung des geteilten beweglichen Stücks in der axialen Richtung kommt.

8. Zahnärztliches Handstück nach Anspruch 4 oder 5,
wobei die geteilten beweglichen Stücke (47A, 47B), das zylindrische Seitenelement (45) und das zylindrische Befestigungselement (57) jeweils aus einem Material hergestellt sind, das in seiner Härte stärker als das elastische Vorspannelement (41) ist.

9. Zahnärztliches Handstück nach Anspruch 4 oder 5,
wobei in einem Querschnitt der Nut ein Verjüngungswinkel, der durch eine Achse der Nut und durch mindestens eine der beiden konischen Oberflächen, entweder durch die zweite konische Oberfläche (53) oder durch die vierte konische Oberfläche (55) von jedem der geteilten beweglichen Stücke (47A, 47B), gebildet ist, in einem Bereich von 5 Grad oder mehr und 30 Grad oder weniger liegt.

10. Zahnärztliches Handstück nach einem der Ansprüche 1 bis 5, das ferner umfasst:
einen Schieber (61), der vor den geteilten beweglichen Stücken (47A, 47B) in der Einsetzrichtung angeordnet ist, und der in einem Fall, in dem das zahnärztliche Behandlungswerkzeug (15) von dem Spannfuttermechanismus gelöst werden soll, nach unten gedrückt wird,
wobei der Schieber ein Schieberstück (63) enthält, das sich in das zylindrische Seitenelement (45) durch einen Umfangsspalt zwischen den mehreren geteilten beweglichen Stücken (47A, 47B) erstreckt, und
wobei in einem Fall, in dem der Schieber (61) nach unten rückwärtig in die Einsetzrichtung des zahnärztlichen Behandlungswerkzeugs (15) gedrückt wird, das Schieberstück (63) das zylindrische Seitenelement (45) rückwärtig zurück in die Einsetzrichtung drückt, so dass das Spannfutter des zahnärztlichen Behandlungswerkzeugs durch die geteilten beweglichen Stücke (47A, 47B) gelöst wird.

11. Zahnärztliches Handstück nach einem der Ansprüche 1 bis 5,
wobei ein Radius der inneren Umfangsoberfläche von jedem der geteilten beweglichen Stücke (47A, 47B) kleiner als ein Radius der äußeren Umfangsoberfläche des zahnärztlichen Behandlungswerkzeugs (15) ist.

12. Zahnärztliches Handstück nach einem der Ansprüche 1 bis 5,
wobei das elastische Vorspannelement eine Spiralfeder (41) enthält.

13. Zahnärztliches Handstück nach einem der Ansprüche 1 bis 5,
wobei jedes der geteilten beweglichen Stücke (47A, 47B) eine symmetrische Form aufweist, in der eine Ebene, die durch eine axiale Mitte verläuft und senkrecht zu der axialen Richtung angeordnet ist, als eine Symmetrieebene gebraucht wird.

## Revendications

1. Pièce à main dentaire (100) configurée pour entraîner un outil de traitement dentaire (15) en rotation monté dans un mécanisme de mandrin, la pièce à main dentaire comprenant :
le mécanisme de mandrin dans lequel l'outil de traitement dentaire est monté de façon à pouvoir être fixé et séparé,
dans laquelle le mécanisme de mandrin inclut :
un manchon cylindrique creux (37) dans lequel l'outil de traitement dentaire (15) est inséré de façon à être supporté en rotation ;
un membre cylindrique coulissant (45) logé dans le manchon de façon à être mobile dans une direction axiale du manchon ;
une pluralité de pièces mobiles divisées (47A, 47B) disposées dans le manchon à différentes positions circonférentielles l'une par rapport à l'autre, chacune de la pluralité de pièces mobiles divisées incluant une surface périphérique extérieure qui vient en contact avec une surface périphérique intérieure du membre cylindrique coulissant, et une surface périphérique intérieure qui vient en contact avec une surface périphérique extérieure de l'outil de traitement dentaire inséré dans le manchon, et l'outil de traitement dentaire étant maintenu entre la pluralité de pièces mobiles divisées ;
un membre de décalage élastique (41) configuré pour décaler le membre cylindrique coulissant vers l'avant dans une direction d'insertion de l'outil de traitement dentaire ; et
une unité de butée (49) configurée pour restreindre le mouvement axial vers l'avant de la pluralité de pièces mobiles divisées dans la direction d'insertion, au moins une partie de l'unité de butée étant fixée partiellement sur le manchon, et
dans laquelle la surface périphérique extérieure de chacune de la pluralité de pièces mobiles divisées (47A, 47B) et la surface périphérique intérieure du membre cylindrique coulissant (45) viennent en contact l'une avec l'autre par le biais d'une portion d'ajustement conique (51, 53) ayant un gradient dans la direction axiale, la portion d'ajustement conique étant prévue sur au moins une de la surface périphérique extérieure de la pluralité de pièces mobiles divisées (47A, 47B) et de la surface périphérique intérieure du membre cylindrique coulissant (45),
dans laquelle les pièces mobiles divisées (47A, 47B) sont déplacées dans une direction radiale par un mouvement relatif entre chacune des pièces mobiles divisées (47A, 47B) et le membre cylindrique coulissant (45) dans la direction axiale, de façon à ce que la fixation de l'outil de traitement dentaire (15) et la libération de la fixation soient réalisées, et
**caractérisé en ce que**
la pluralité de pièces mobiles divisées (47A, 47B) sont disposées dans le manchon (37) de façon à pouvoir tourner par rapport au manchon sur un axe du manchon.

2. Pièce à main dentaire selon la revendication 1, dans laquelle une paire des pièces mobiles divisées (47A, 47B) sont disposées de façon à se faire face l'une l'autre dans une direction diamétrale du manchon (37).

3. Pièce à main dentaire selon la revendication 1,
dans laquelle le membre cylindrique coulissant (45) inclut, sur la surface périphérique intérieure du membre cylindrique coulissant, une première surface conique (51) dont le diamètre augmente vers l'avant dans la direction d'insertion, et
dans laquelle chacune de la pluralité de pièces mobiles divisées (47A, 47B), inclut, sur la surface périphérique extérieure de la pièce mobile divisée, une deuxième surface conique (53) qui est configurée pour venir en contact de coulissement avec la première surface conique.

4. Pièce à main dentaire selon la revendication 3,
dans laquelle l'unité de butée (49) inclut :
un membre de fixation cylindrique (57) qui est disposé à une position plus en avant que la pluralité de pièces mobiles divisées (47A, 47B) dans la direction d'insertion, et qui inclut, sur une surface périphérique intérieure du membre de fixation cylindrique, une troisième surface conique (55) dont le diamètre est réduit vers l'avant dans la direction d'insertion ; et
une quatrième surface conique (59) qui est prévue sur la surface périphérique extérieure de chacune de la pluralité de pièces mobiles divisées (47A, 47B) et qui est configurée pour venir en contact de coulissement avec la troisième surface conique.

5. Pièce à main dentaire selon la revendication 4,
dans laquelle le membre de fixation cylindrique (57) est divisé, dans la direction axiale, en un membre cylindrique conique dans une région incluant la troisième surface conique, et un membre cylindrique restant dans une région excluant le membre cylindrique conique.

6. Pièce à main dentaire selon l'une quelconque des revendications 1 à 5,
dans laquelle chacune des pièces mobiles divisées (47A, 47B) est dotée, sur la surface périphérique extérieure vers l'avant de la partie d'ajustement conique dans la direction d'insertion, d'une partie en saillie (81) qui fait saillie vers l'extérieur dans la direction radiale, et
dans laquelle la partie en saillie peut être en contact avec une partie d'extrémité avant du membre coulissant cylindrique (45) dans la direction d'insertion.

7. Pièce à main dentaire selon la revendication 4 ou 5, comprenant en outre :
un anneau de restriction annulaire (65) qui est disposé sur une partie d'extrémité arrière du membre de fixation cylindrique (57) dans la direction d'insertion et qui est fixé au manchon (37),
dans laquelle dans chacune des pièces mobiles divisées (47A, 47B), une rainure en creux (67) est formée le long d'une direction circonférentielle sur une surface de diamètre extérieur entre la deuxième surface conique (53) et la quatrième surface conique (55), et
dans laquelle l'anneau de restriction présente une épaisseur radiale telle qu'une surface périphérique intérieure de la bague de restriction est insérée dans la rainure de réception, et une paroi de rainure avant ou une paroi de rainure arrière de la rainure en creux dans la direction d'insertion vient en contact avec l'anneau de restriction par le mouvement de la pièce mobile divisée dans la direction axiale.

8. Pièce à main dentaire selon la revendication 4 ou 5,
dans laquelle les pièces mobiles divisées (47A, 47B), le membre coulissant cylindrique (45) et le membre de fixation cylindrique (57) sont faits chacun dans un matériau qui a une dureté supérieure au membre de décalage élastique (41).

9. Pièce à main dentaire selon la revendication 4 ou 5,
dans laquelle dans une section transversale axiale du manchon, un angle de conicité formé par un axe du manchon et au moins une de la deuxième surface conique (53) et de la quatrième surface conique (55) de chacune des pièces mobiles divisées (47A, 47B) est de 5 degrés ou plus et de 30 degrés ou moins.

10. Pièce à main dentaire selon l'une quelconque des revendications 1 à 5, comprenant en outre :
un poussoir (61) qui est disposé à l'avant des pièces mobiles divisées (47A, 47B) dans la direction d'insertion et qui est appuyé vers le bas dans un cas où l'outil de traitement dentaire (15) doit être séparé du mécanisme de mandrin,
dans laquelle le poussoir inclut une poussette (63) qui s'étend vers le membre coulissant cylindrique (45) à travers un espace circonférentiel entre la pluralité de pièces mobiles divisées (47A, 47B), et
dans laquelle, dans un cas où le poussoir (61) est appuyé vers le bas vers l'arrière dans la direction d'insertion de l'outil de traitement dentaire (15), la poussette (63) repousse le membre coulissant cylindrique (45) vers l'arrière dans la direction d'insertion, de façon à ce que la tenue par mandrin de l'outil de traitement dentaire par les pièces mobiles divisées (47A, 47B) soit libérée.

11. Pièce à main dentaire selon l'une quelconque des revendications 1 à 5,
dans laquelle un rayon de la surface périphérique intérieure de chacune des pièces mobiles divisées (47A, 47B) est plus petit qu'un rayon de la surface périphérique extérieure de l'outil de traitement dentaire (15).

12. Pièce à main dentaire selon l'une quelconque des revendications 1 à 5,
dans laquelle le membre de décalage élastique inclut un ressort hélicoïdal (41).

13. Pièce à main dentaire selon l'une quelconque des revendications 1 à 5,
dans laquelle chacune des pièces mobiles divisées (47A, 47B) présente une forme symétrique dans laquelle un plan passant à travers un centre axial et perpendiculaire à la direction axiale est utilisé en tant qu'un plan de symétrie.
